# EUROPEAN PATENT SPECIFICATION

(11) **EP 4 091 647 B1**
(45) Date of publication and mention of the grant of the patent: **01.10.2025**
(21) Application number: 21174181.4
(22) Date of filing: 17.05.2021
(51) Int. Cl.: A61M 1/28

(54) **DIALYSIS CASSETTE WITH PUMP**
DIALYSEKASSETTE MIT PUMPE
CASSETTE DE DIALYSE COMPORTANT UNE POMPE

(43) Date of publication of application: 23.11.2022
(73) Proprietor: Bellco S.r.l., 41037 Mirandola (Modena) (IT)
(72) Inventor: MARRA, Antonio Giuseppe, 41037 Mirandola (Modena) (IT); ORLANDINI, Salvatore, 41037 Mirandola (Modena) (IT); PAGATINI, Guilherme, 41037 Mirandola (Modena) (IT); PETRUCCI, Alberto, 41037 Mirandola (Modena) (IT); MAROTTA, Gaspare, 41037 Mirandola (Modena) (IT)
(74) Representative: Greaves Brewster LLP

(56) References cited:
- EP-A1- 3 991 770
- US-A1- 2005 209 563
- US-A1- 2018 010 612

## Description

### FIELD

The disclosure relates to a cassette for a dialysis system for managing fluids for generating one or more dialysis fluid solutions. The cassette can incorporate a flexible rotor and can optionally, integrate the flexible rotor or volumetric pump inside the cassette body. The cassette can include a flexible vane pump that provides for precise volumetric control of an amount of dialysis fluid or any other fluid pumped by each rotation of the pump vane or rotor.

### BACKGROUND

Conventional dialysis machines often use a pumping cassette to direct and control the movement of fluids. The conventional pumping cassette typically include a flexible membrane that is moved mechanically to create a positive and negative pressure to move fluid out of and into, respectively, the cassette. Certain known systems include flexible sheeting on one side of the cassette, while others include sheeting on both sides of the cassette. Positive and/or negative pressure can be used to operate the pumping cassettes. However, the conventional pumping cassettes can be inaccurate or imprecise. The cassettes may not accurately and/or precisely measure volumes of fluid being pumped by the cassette. Hence, there is a need for systems and methods that can monitor and provide volumetric control of a fluid being pumped. The need includes a cassette that can have an external rotary vane pump. The need still further includes a cassette having an integrated pump. The need includes a cassette having an integrated rotary pump. The need includes a rotary pump having flexible vanes. The need still also includes controlling a defined volume of fluid being pumped or moved per each rotation of the pump vanes.

Relevant prior art is for instance disclosed in documents US2018/010612 A1 and US2005/209563 A1.

### SUMMARY OF THE INVENTION

The problem to be solved is a fluid cassette for use with a dialysis system having precise and accurate volumetric control over pumped fluids. The solution is to include a flexible rotary pump either exterior or integrated into a cassette that moves a defined amount of fluid per each rotation of the pump vane. The solution can be incorporated into cassettes for use with peritoneal systems, hemodialysis systems, and any other fluid therapy or replacement systems.

A dialysis cassette according to the present invention comprises the technical features as defined in independent claim 1. A dialysis system according to the present invention comprises the technical features as defined in claim 10.

The first aspect relates to a dialysis cassette. According to the invention as claimed, the dialysis cassette has a cassette housing having any number of fluid channels and a pump assembly inside the cassette housing, the pump assembly also having a pump housing and a flexible rotor having any number of flexible vanes, wherein the flexible rotor is rotatable in either a clockwise direction or a counterclockwise direction to move a defined amount of fluid through any number of channels per each rotation of the flexible vanes.

In any embodiment, the pump housing can have an inlet and an outlet coupled to the any number of channels.

In any embodiment, the pump housing can have a narrowing portion disposed between the inlet and the outlet.

In any embodiment, the plurality of flexible vanes is configured to compress when contacting the narrowing portion.

In any embodiment, the flexible rotor is comprised of an elastomer and a thermoplastic polymer.

In any embodiment, the flexible rotor is rotatable about a rotation axis perpendicular to a plane defined by the cassette housing.

In any embodiment, the pump housing can have a drive shaft and a gasket disposed about the drive shaft, the gasket configured to fluidly seal the pump housing.

Each valve of any number of valves is a pinch valve having an undulating shape with a deflectable portion.

In any embodiment, the cassette housing further can have at least one sensor disposed in fluid contact with any number of channels.

In any embodiment, the at least one sensor is at least one of a pressure sensor, a temperature sensor, a conductivity sensor, an air bubble detector, or a flow sensor.

The second aspect relates to a system. The dialysis system has a dialysis cycler, a cassette interface, a patient line, a drain line; and a fluid source; and a cassette that can couple to the cassette interface, the patient line, the drain line, and the fluid source, the cassette having any number of channels fluidly connectable to any number of connectors each of which can be coupled to a respective one of the patient line, the drain line, and the fluid source can have any number of valves disposed within any number of channels; and a pump assembly disposed within the cassette housing, the pump assembly having a pump housing and a flexible rotor having any number of flexible vanes, wherein the flexible rotor is rotatable in either a clockwise direction or a counterclockwise direction to move a defined amount of fluid through any number of channels per each rotation of the flexible vanes.

In any embodiment, the pump housing can have an inlet and an outlet coupled to any number of channels.

In any embodiment, the pump housing can have a narrowing portion positioned between the inlet and the outlet.

In any embodiment, any number of flexible vanes can be configured to compress when contacting the narrowing portion.

In any embodiment, the flexible rotor can be made of an elastomer and a thermoplastic polymer.

In any embodiment, the flexible rotor is rotatable about a rotation axis perpendicular to a plane defined by the cassette housing.

In any embodiment, the pump housing can have a drive shaft and a gasket disposed about the drive shaft, the gasket configured to fluidly seal the pump housing.

Each valve of any number of valves is a pinch valve having an undulating shape with a deflectable portion.

In any embodiment, the cassette interface can have a plurality of actuators configured to push on the deflectable portion.

In any embodiment, the cassette housing further can have at least one sensor disposed in fluid contact with any number of channels, the at least one sensor being one of a pressure sensor, a temperature sensor, a conductivity sensor, an air bubble detector, or a flow sensor.

### BRIEF DESCRIPTION OF THE DRAWINGS

FIG. 1 is a perspective view of a dialysis system including a cycler and a fluid cassette.
FIG. 2 is a perspective view of the cycler of FIG. 1.

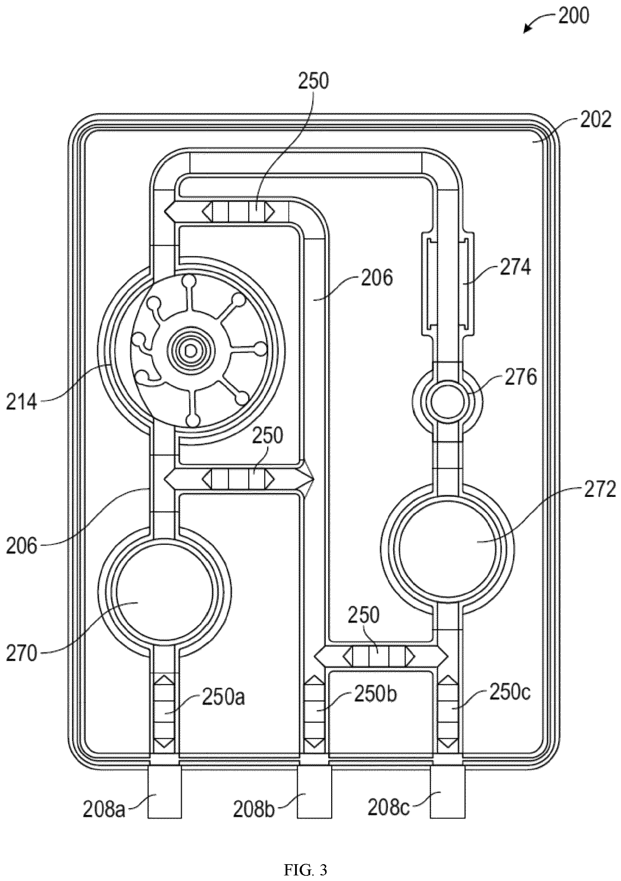

FIG. 3 is a partially exposed, plan view of an inner portion of the cassette of the PD system of FIG. 1.

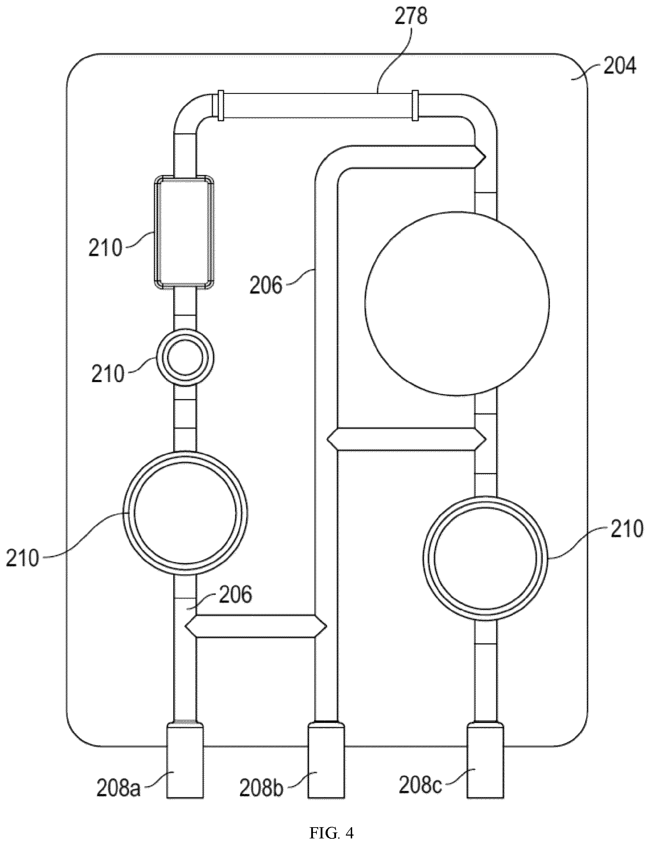

FIG. 4 is a plan view of an outer portion of the cassette of FIG. 3.

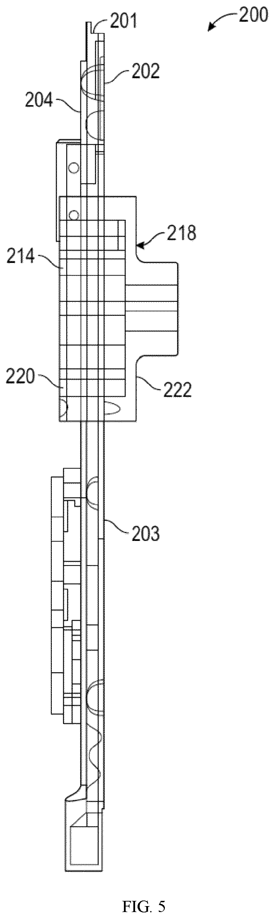

FIG. 5 is a side view of the cassette of FIG. 3.

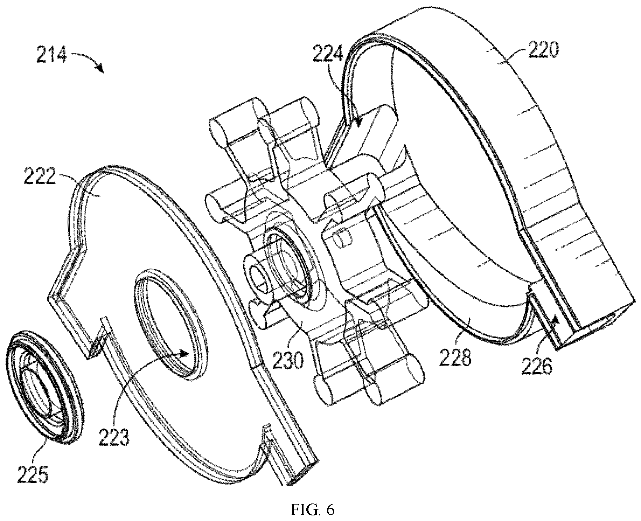

FIG. 6 is an exploded, perspective view of a pump assembly of the cassette of FIG. 3.
FIG. 7 is a cross-sectional view of the pump assembly of FIG. 6.
FIG. 8 is a perspective view of a rotor of the pump assembly of FIG. 6.
FIG. 9 is a perspective view of a drive shaft for rotating the rotor of FIG. 8.
FIG. 10 is a perspective view of a pinch valve of the cassette of FIG. 3.

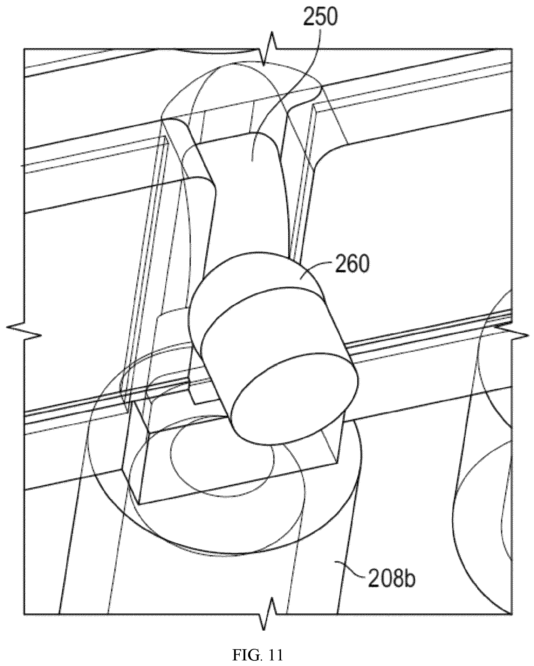

FIG. 11 is a perspective view of an actuator for actuating the pinch valve of FIG. 10.
FIG. 12 is a plan view of the cassette of FIG. 3 in a pump priming configuration.
FIG. 13 is a plan view of the cassette of FIG. 3 in a filling configuration.
FIG. 14 is a plan view of the cassette of FIG. 3 in a draining configuration.

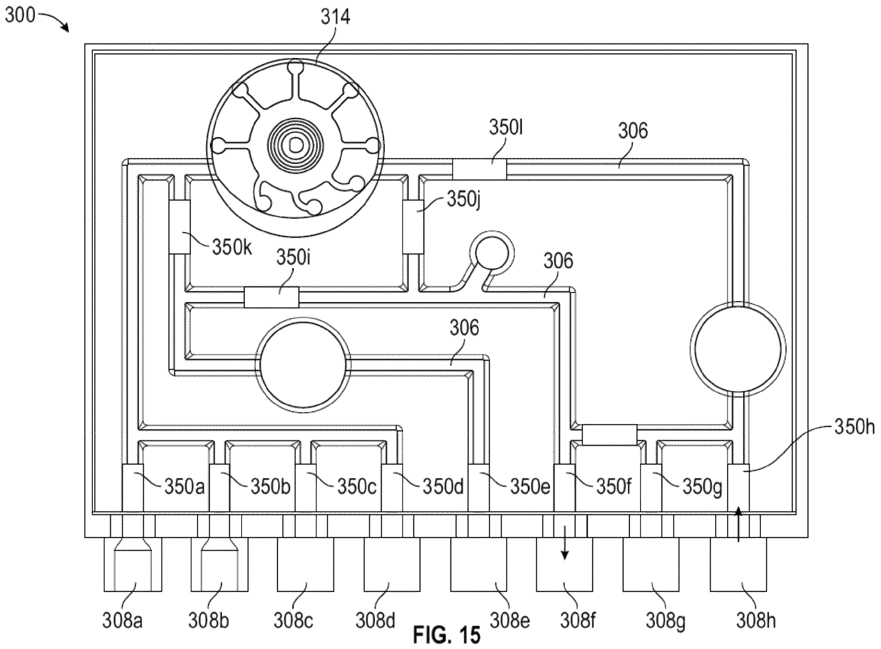

FIG. 15 is a partially exposed, plan view of an inner portion of a cassette.
FIG. 16 is a plan view of the cassette of FIG. 15 in a bag filling configuration.
FIG. 17 is a plan view of the cassette of FIG. 15 in a pump priming configuration.
FIG. 18 is a plan view of the cassette of FIG. 15 in a filling configuration.
FIG. 19 is a plan view of the cassette of FIG. 15 in a draining configuration.

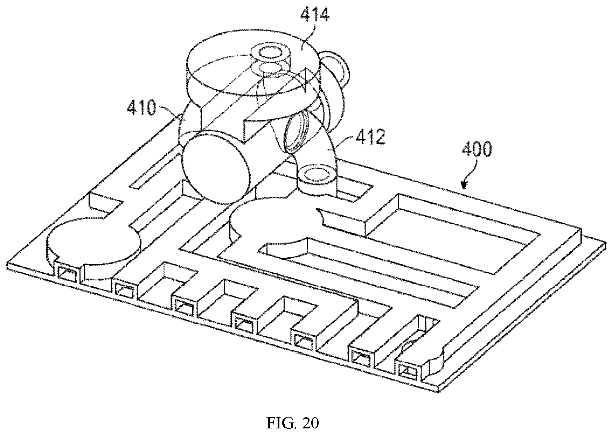

FIG. 20 is a perspective view of a cassette in any embodiment

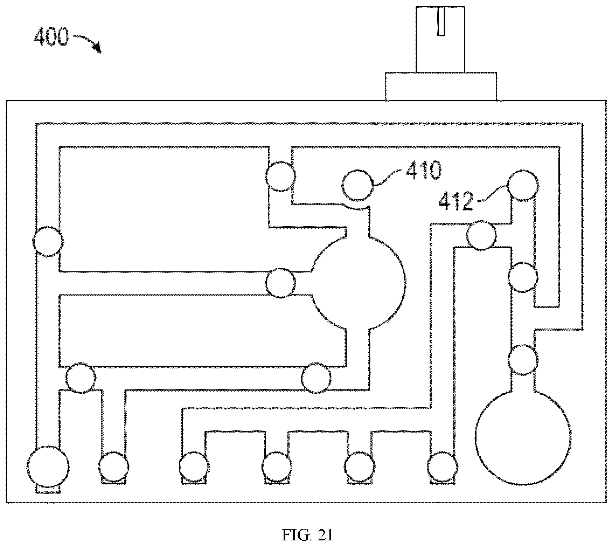

FIG. 21 is a cross-sectional, plan view of the cassette of FIG. 20.

### DETAILED DESCRIPTION

Unless defined otherwise, all technical and scientific terms used have the same meaning as commonly understood by one of ordinary skill in the art.

The articles "a" and "an" are used to refer to one to over one (i.e., to at least one) of the grammatical object of the article. For example, "an element" means one element or over one element.

The term "comprising" includes, but is not limited to, whatever follows the word "comprising." Use of the term indicates the listed elements are required or mandatory but that other elements are optional and can be present.

The term "consisting of" includes and is limited to whatever follows the phrase "consisting of." The phrase indicates the limited elements are required or mandatory and that no other elements can be present.

The term "consisting essentially of" includes whatever follows the term "consisting essentially of" and additional elements, structures, acts or features that do not affect the basic operation of the apparatus, structure or method described.

A "dialysis cassette" refers to a set of components physically connected that are used in peritoneal dialysis therapy.

The term "disposable" refers to a component or set of components intended for use a single time, or a set amount of times and then replaced.

The term "flow sensor" refers to any component capable of measuring a volume or a rate of fluid moving through a conduit.

The term "fluid" can be any substance without a fixed shape that yields easily to external pressure such as a gas or a liquid. Specifically, the fluid can be water, preferably purified water, and can be water containing any solutes at any concentration. The fluid can be used as a solvent (to dissolve one or more solutes). The fluid can also be dialysate of any type including fresh, partially used, or spent. The fluid can also contain one or more dissolved gas. The fluid can be blood.

A "fluid line" is any conduit through which fluid can move.

The term "fluid flow path" or "flow path" refers to any set of conduits or components that allow for the movement of fluids or gases.

The term "fluidly connectable" refers to the ability to provide passage of fluid from one point to another point. The ability to provide such passage can be any mechanical connection, fastening, or forming between two points to permit the flow of fluid, gas, or combinations thereof. The two points can be within or between any one or more of compartments, modules, systems, components, and rechargers, all of any type. Most preferably the connections are provided by connectors, fluid passage is provided by tubes or pipes.

The term "fluidly connected" refers to a particular state or configuration of one or more components such that fluid can flow from one point to another point. The connection state can also include an optional unconnected state or configuration, such that the two points are disconnected from each other to discontinue flow. It will be further understood that the two "fluidly connectable" points, as defined above, can from a "fluidly connected" state. The two points can be within or between any one or more of compartments, modules, systems, components, and rechargers, all of any type.

"Peritoneal dialysis" is a therapy wherein a dialysate is infused into the peritoneal cavity, which serves as a natural dialyzer. In general, waste components diffuse from a patient's bloodstream across a peritoneal membrane into the dialysis solution via a concentration gradient. In general, excess fluid in the form of plasma water flows from a patient's bloodstream across a peritoneal membrane into the dialysis solution via an osmotic gradient. Once the infused peritoneal dialysis solution can have captured sufficient amounts of the waste components the fluid is removed. This cycle can be repeated for several cycles each day or as needed.

A "peritoneal dialysis cycler" or "PD cycler" is a component or set of components for movement of fluid into and out of the peritoneal cavity of a patient

"Peritoneal dialysis fluid" is a fluid solution to be used in peritoneal dialysis having specified parameters for purity and sterility and containing one or more solutes. Peritoneal dialysis fluid is different than the dialysate used in hemodialysis.

A "peritoneal dialysis system" is a set of components capable of performing at least one function for peritoneal dialysis therapy.

The term "plurality" can mean any one or more of a component or a thing.

### Cassettes

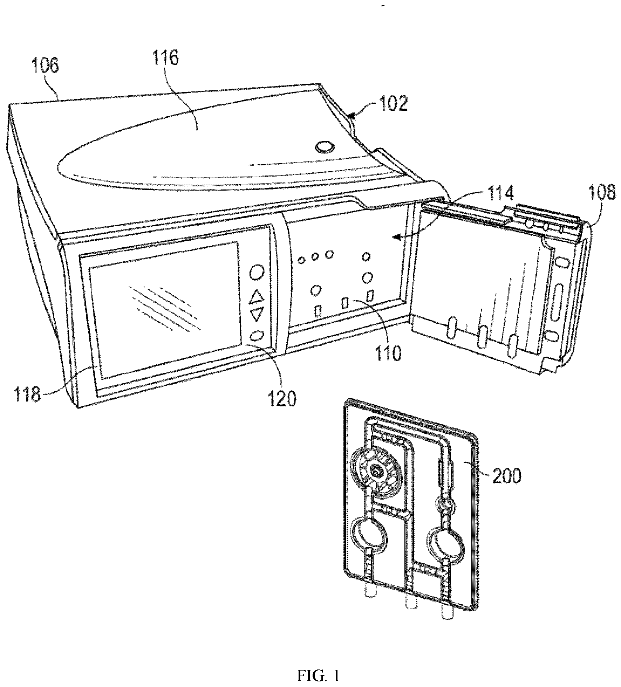

FIG. 1 shows a dialysis system 100 having a cycler 102 and a fluid cassette 200. However, the systems, methods, and components are applicable to any medical fluid therapies such as peritoneal dialysis (including APD, CAPD, CFPD and tidal flow modalities), hemodialysis, hemofiltration, hemodiafiltration, any type of continuous renal replacement therapy, congestive heart failure therapy as well as others. In any embodiment, a flexible vane rotor pump can be incorporate for precise and/or accurate volumetric control. Each rotation of a rotor pump can contain a defined volume of fluid. In any embodiment, the systems, methods, and components can have suitable tubing for pumping, an exterior or integrated rotary pump, valving for cassette-based systems, air detection and compensation, and one or more controllers. In any embodiment, the cycler **102** and the cassette **200** can be used to provide continuous cycler-assisted dialysis. The cycler **102** can be used in a home environment. The cycler **102** can have a size and weight suitable for home use. The cycler **102** can be supported on a cart **104** that is used to improve ease of handling and storage of the cycler **102.** The cycler **102** can have a housing **106,** a door **108,** and a cassette interface **110** that abuts an inner portion **202** of the cassette **200** when the cassette **200** is disposed within a cassette compartment **114** formed between the cassette interface **110** and the closed door **108.** A heater tray **116** can be positioned on top of the housing **106.** The heater tray **116** can be sized and shaped to accommodate a bag of dialysis solution (e.g., a 5-liter bag of solution). The cycler **102** also includes a display screen **118** and control buttons **120.** In embodiments, the display screen **118** can be a touchscreen. The display screen **118** and control buttons **120** can be operated by a user (e.g., a patient) to allow, for example, set-up, initiation, and/or termination of a PD treatment.

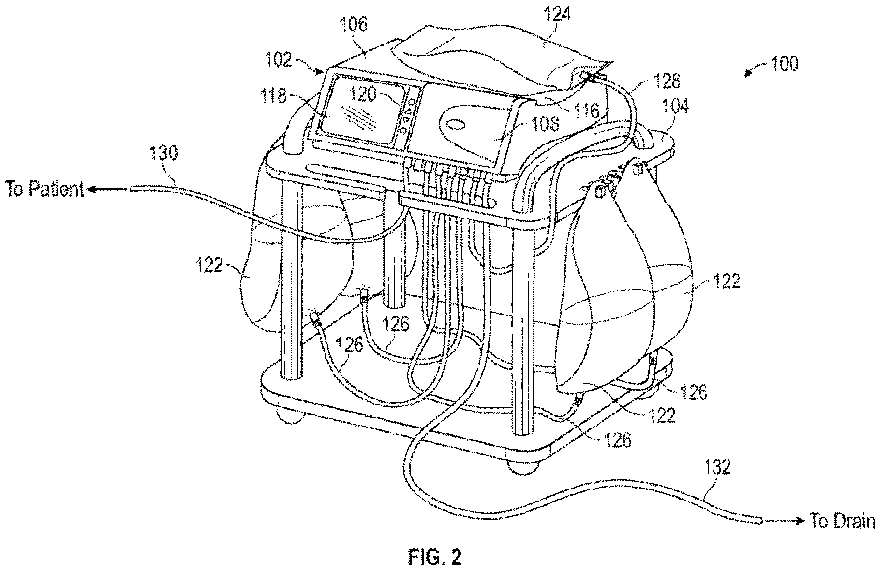

Dialysis solution storage bags **122** can be suspended from the sides of the cart **104,** and a dialysis solution fill bag **124** can be positioned on the heater tray **116,** as shown in FIG. 2. The storage bags **122** and the fill bag **124** are connected to the cassette **200** via storage bag lines **126** and a fill bag line **128,** respectively. The storage bag lines **126** can be used to pass dialysis solution from storage bags **122** to the cassette **200** during use, and the fill bag line **128** can be used to pass dialysis solution back and forth between the cassette **200** and the fill bag **124** during use. In addition, a patient line **130** and a drain line **132** are connected to the cassette **200.** The patient line **130** can be connected to a patient's abdomen via a catheter and can be used to pass dialysis solution back and forth between the cassette **200** and the patient during use. The drain line **132** can be connected to a drain or drain receptacle and can be used to pass dialysis solution from the cassette **200** to the drain or drain receptacle during use.

In FIG.'s 3-5, the cassette **200** can have a cassette housing **201** formed from an inner portion **202** and an outer portion **204.** The entirety or a segment of the inner portion **202** can be formed from single or multiple layer flexible membrane **203.** The flexible membrane **203** can be constructed from PVC, PP, silicon rubber, EPDM, or like materials. The inner portion **202** contacts the cassette interface **110** once the cassette **200** is loaded into the cassette compartment **114** of the cycler **102.** The outer portion **204** can be formed from a rigid material and contacts the door **108** once the cassette **200** is loaded into the cassette compartment **114** of the cycler **102.** The outer portion **204** can be formed from a thermoplastic polyester including, but not limited to, polyethylene terephthalate glycol, PVC, PETG, high-density polyethylene, and combinations thereof. The outer portion **204** can be transparent. The outer portion **204** defines a plurality of fluid channels **206,** which are fluidly coupled to a plurality of fluid line connectors **208a, 208b, 208c,** which act as inlet/output ports of the cassette **200.**

The fluid channels **206** can be fluidly coupled to a plurality of sensor portions **210,** which are larger than the fluid channels **206** and can be of various shapes (e.g., cylindrical, circular, etc.) to accommodate sensors within the cassette **200.** As shown in FIG.'s 3-5, the cassette **200** includes a plurality of sensors, namely, a first pressure sensor **270,** a second pressure sensor **272,** a conductivity sensor **274,** a temperature sensor **276,** and an air bubble detector **278.** Although shown in different locations in FIG.'s 3-5, any of the sensors can be integrated into a single position. For example, conductivity sensor **274** and temperature sensor **276** can be in the same position. In certain embodiments, the pressure sensor **270,** pressure sensor **272,** temperature sensor **276,** and/or air bubble detector **278** can be contactless sensors that do not have direct contact with the fluid in the fluid channels **206.** These sensors are used to monitor pressure, temperature, conductivity, and presence of air bubbles in the dialysis fluid, respectively. Pressure sensors **270** and **272** can be pressure transducers. Conductivity sensor **274** can include two pairs of electrodes configured to determine conductivity by applying an electric current to a first pair of electrodes, while measuring the current through a second pair of electrodes. Conductivity can be calculated based on measured current using the distance, surface area, and resistance of the electrodes. Temperature sensor **276** can be a thermocouple, IR contactless or any other suitable temperature sensor. The air bubble detector **278** can be an acoustic and/or an optical bubble detector and is configured to detect the presence of air bubbles in the fluid based on disruption in the acoustic and/or optical signals transmitted through the fluid. In any embodiment one or more flow sensors can also be included. The flow sensor can be an optical sensor to measure a flow rate of fluid through the fluid channels **206.**

The cassette **200** includes a plurality of valves **250a-f** disposed within fluid channels **206,** which direct dialysis solution through the cassette **200.** Although shown as six valves in FIG.'s 3-5, any number of vales can be included in the cassette **200.** The cassette **200** can include 7, 8, 9, 10, or more valves. In certain embodiments, the cassette **200** can include up to 13 valves. The cassette **200** also includes an integrated pump assembly **214** having a pump housing **218.** The pump housing **218** can include a first pump housing portion **220** integrally formed as part of the outer portion **204** and a second pump housing portion **222** extending from the inner portion **202.** During operation, the dialysis solution flows through the pump assembly **214** and the flow depends on the speed and direction of the pump assembly **214** as well as the state of the valves **250a-f.**

Fluid line connectors **208a-g** are positioned along the bottom edge of the cassette **200.** As noted above, the fluid channels **206** in the cassette **200** lead from the pump assembly **214** to the connectors **208a-g.** The connectors **208a-g** are configured to fluidly connect to fluid lines and various parts of the PD system. As illustrated in FIG.'s 3-5, the connectors **208a-g** receive fittings of the fill bag line **128,** the patient line **130,** and the drain line **132.** In particular, the fill bag line **128** can be connected to the connector **208a,** the drain line **132** can be connected to the connector **208b,** and the patient line **130** can be connected to the connector **208c.** Flow through each of the connectors **208a- g** can be controlled by a corresponding valve **250a-c.**

In FIG.'s 6 and 7, the pump assembly **214** includes the pump housing **218,** which is formed from the first and second pump housing portions **220** and **222.** The pump housing **218** can be fluidly sealed by coupling the pump housing portions **220** and **222** using adhesive, welding, or using any other suitable methods. In embodiments, the pump housing **218** can be formed as a single component unit via molding. The pump housing **218** also includes a first connection **224** (e.g., inlet) and a second connection **226** (e.g., outlet) formed by the first and second pump housing portions **220** and **222.** The pump assembly **214** can be operated in unidirectional or bidirectional manner such that the first connection **224** is the outlet and the second connection **226** is the inlet. Each of the first and second connections **224** and **226** are coupled to the fluid channels **206** to allow for movement of fluid therethrough by the pump assembly **214.**

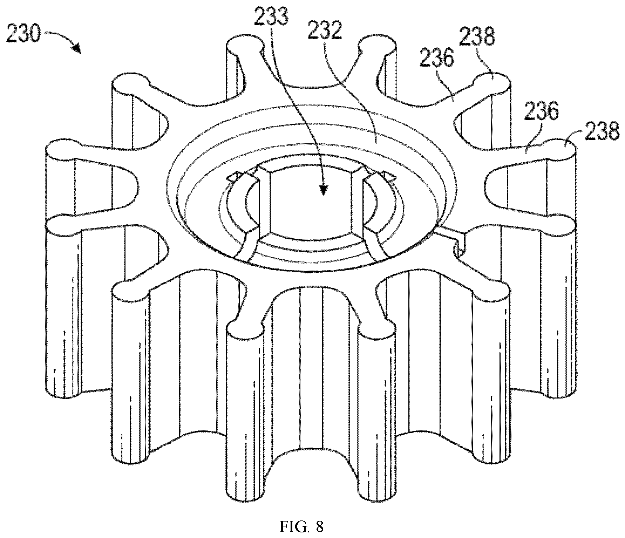

The pump assembly **214** also includes a flexible rotor **230.** In FIG. 8, the flexible rotor **230** includes a central rigid shaft **232** with a plurality of vanes **236** extending radially from the shaft **232.** Each of the vanes **236** terminates in a projection **238,** which can have a cylindrical shape whose longitudinal axis is perpendicular to a radial axis defined by each of the vanes **236.** The flexible rotor **230** can have any number of vanes **236,** which for example can be from 3 to 15. The distance between the vanes **236,** i.e., a straight line between neighboring projections **238,** can be equal to the width or diameter of each of the first and second connections **224** and **226.**

The vanes **236** have substantially the same width as the inner width of the pump housing **218,** such that the vanes **236** contact the inner portion of the pump housing **218.** The flexible rotor **230** can be formed from any suitable flexible polymeric material and can be formed by co-molding an elastomer, such as silicone rubber, and a thermoplastic polymer, including, but not limited to, polyoxymethylenes, polyurethanes, polycarbonate resins, polyamides, polyphenylene sulfides, acrylonitrile butadiene styrene resins, polyether ether ketones, polyphenylene oxides, polypropylenes, and combinations thereof.

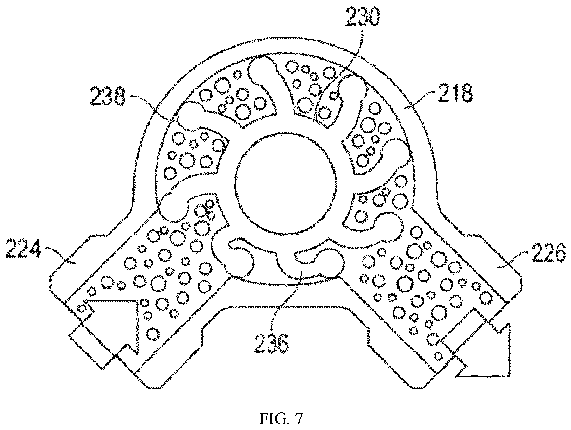

In FIG.'s 6 and 7, the pump housing **218** can have a substantially circular planar cross-section. The pump housing **218** also includes a radial narrowing portion **228,** which reduces the radius of the pump housing **218.** The flexible rotor **230** can be rotated about a rotation axis that is perpendicular to a plane defined by the cassette housing **201,** i.e., the plane between the inner portion **202** and the outer portion **204.** As used herein, the terms "parallel" and "perpendicular" include relative configurations that are substantially parallel and substantially perpendicular up to about + or - 10 degrees from true parallel and true perpendicular. As the flexible rotor **230** is rotated within the pump housing **218,** the vanes **236** are compressed as shown in FIG. 7. The narrowing portion **228** can be disposed between the first and second connections **224** and **226** and can be arcuately-shaped with a larger radius than the radius of the pump housing **218** with the center of the narrowing portion **228** being offset from the center of the pump housing **218.**

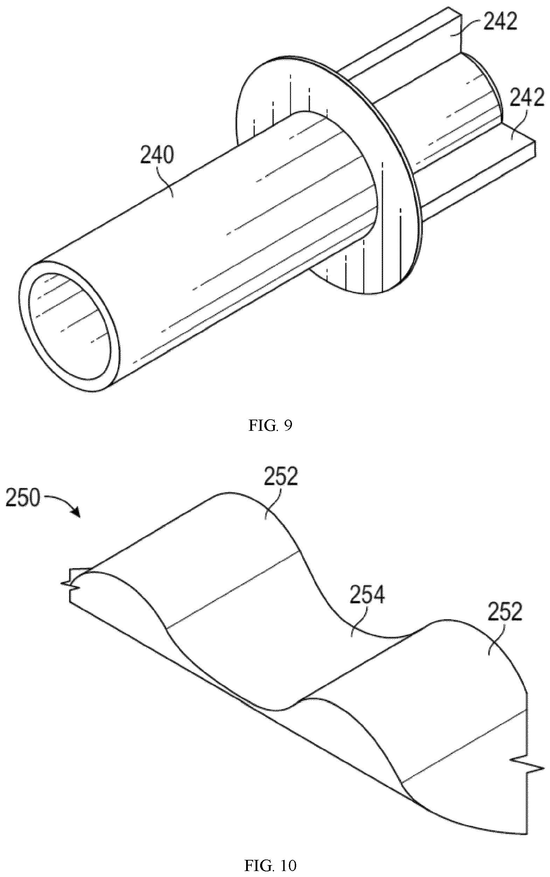

The second pump housing portion **222** defines an opening **223** for coupling the rotor **230** to the cassette interface **110.** In FIG. 9, the pump assembly **214** includes a drive shaft **240** configured to couple to and be rotatable by a motor (not shown). The drive shaft **240** includes a plurality of fins **242** configured to engage a corresponding opening **233** within the central shaft **232** of the flexible rotor **230.** The drive shaft **240** can be inserted through the opening **223.** A gasket **225** covers the opening **223** and forms a fluid-tight seal between the flexible rotor **230** and the drive shaft **240** passing through an opening in the gasket **225.** The gasket **225** can be formed from a polymeric flexible material, which can be formed by co-molding an elastomer, such as silicone rubber, and a thermoplastic polymer, such as, polyester, polyethylene terephthalate glycol, high-density polyethylene, and combinations thereof.

In FIG.'s 10 and 11, the valves **250a-f,** shown as a valve **250** can be a pinch valve formed from the same material as the gasket **225.** The valve **250** is disposed within the fluid channel **206** and a bottom surface of the valve **250** abuts the flexible membrane **203.** The valve **250** has an undulating shape having two peak portions **252** and a deflectable valley portion **254,** such that the valley portion **254** is thinner than the adjacent peak portions **252.** The valve **250** can be actuated by an actuator **260** disposed within the cassette interface **110.** The actuator **260** can be a spring-loaded pneumatic or solenoid actuator. The actuator **260** can be configured to move longitudinally, thereby pushing the valley portion **254** of the valve **250.** Since the surface of the valve **250** abuts the flexible membrane **203,** the actuator **260** acts on the deflectable valley portion **254** through the flexible membrane **203.** Due to the flexible nature of the valve **250,** the valley portion **254** is movable vertically between the peaks **252** from an open configuration, as shown in FIG. 10, to a closed configuration, in which the valley portion **254** blocks fluid passage through the fluid channels **206** in response to the movement of the actuator **260.** Thus, the flow of dialysis solution through the cassette **200** can be controlled through the selective depression of the valley portion **254** by selectively activating the actuators **260** and rotating the rotor **230** in either direction, i.e., clockwise or counterclockwise, at a selected rate of rotation.

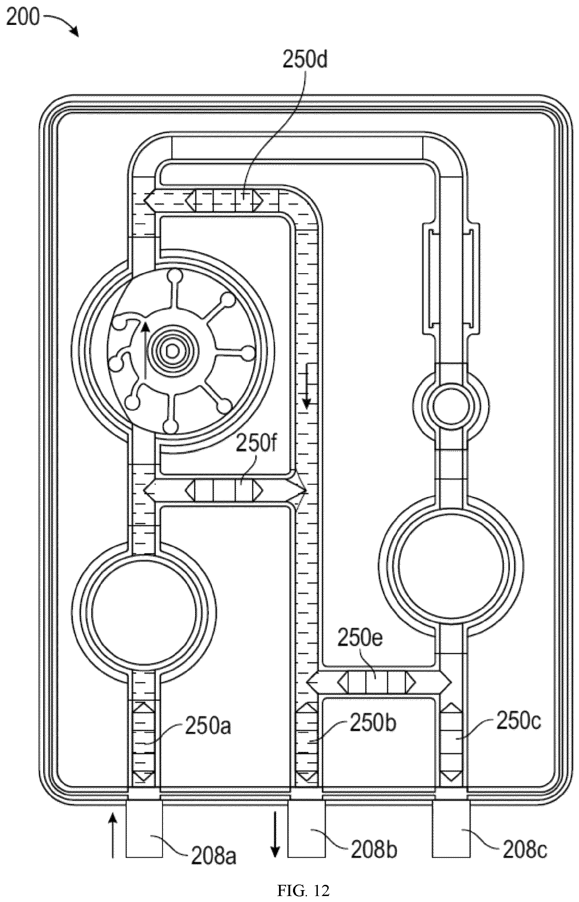

FIG. 12 shows the cassette **200** in a priming configuration in which valves **250a, b,** and **d** are open and the pump assembly **214** is operated in a first direction. In this configuration, the dialysis solution flows from the fill bag line **128** connected to the connector **208a** to the connector **208b** and to the drain line **132.** The remaining valves **250c, e,** and **f are** closed. In this configuration, the fluid channels **206** interconnecting the connectors **208a** and **208b** are primed.

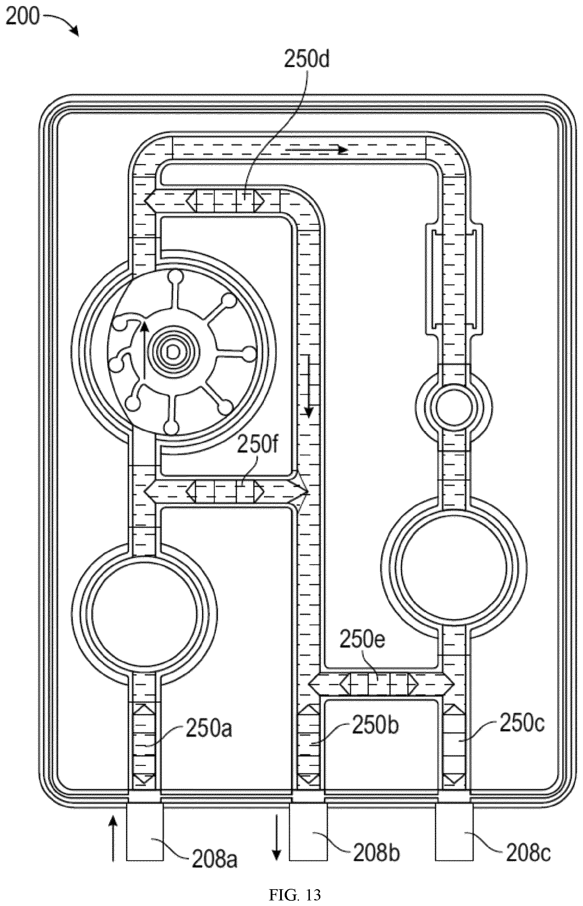

In FIG. 13, the cassette **200** is shown in a patient line priming and filling configuration in which the valves **250a, b, d,** and e are open and the pump assembly **214** is operated in the first direction to move the fluid to the drain line **132.** In this configuration, a portion of the fluid channels **206** are primed. Once all of the selected channels are primed, the valves **250b, d, e,** and **f** are closed and the valve **250c** can be opened, such that fluid from the fill bag line **128** connected to the connector **208a** flows to the connector **208c** and to the patient line **130** to allow for the dialysis solution to be infused into the patient.

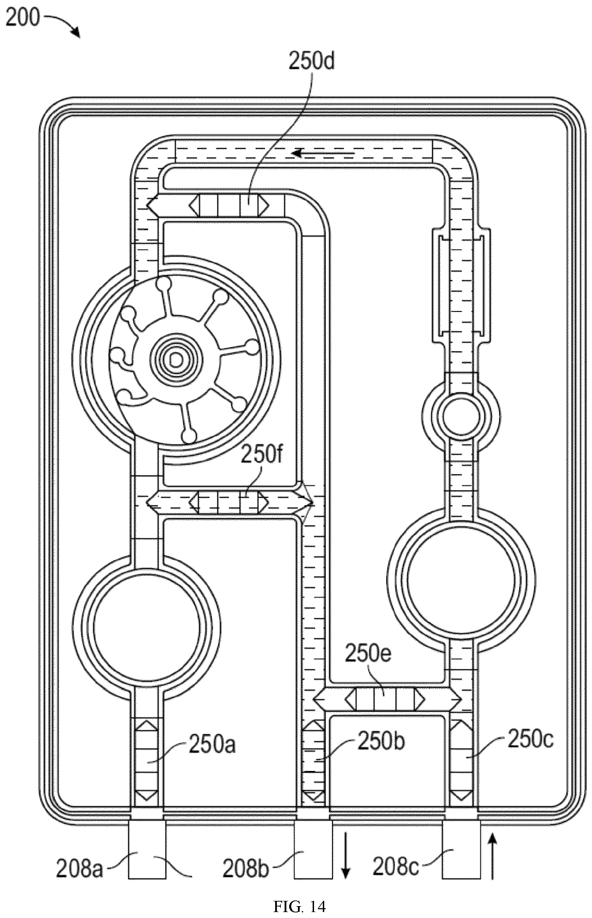

FIG. 14 shows the cassette **200** in a drain configuration, in which the dialysis solution can be removed from the patient after the infusion is completed and the dialysis solution can have dwelled in the patient for the duration of the treatment time. In this configuration, the valves **250a, e, d** are closed and the valves **250b, c, f** are open. The pump assembly **214** can be operated in a second direction, reverse of the first direction, such that the dialysis solution is drained from the patient. In this configuration, the dialysis solution can be withdrawn from the patient through the patient line **130** connected to the connector **208c** and supplied to the connector **208b** coupled to the drain line **132.**

In FIG.'s 15-19, a cassette **300** similar to the cassette **200** is shown. The cassette **300** can include the pump assembly **314.** One difference between the cassette **200** and cassette **300** can be a plurality of connectors **308a-h** for coupling to a plurality of fluid sources. In particular, the connectors **308a-d** can be configured to couple to the storage bag line **126.** The connector **308e** can be configured to couple to the fill bag line **128.** The connector **308f** can be configured to couple to the drain line **132,** and the connectors **308g** and **h** are configured to couple to one or more patient lines **130.** The cassette **200** can be configured to move the dialysis solution between the fill bag **124** and one or more of the storage bags **122.** The cassette **300** includes a plurality of channels **306** with valves **350a-l** disposed therein.

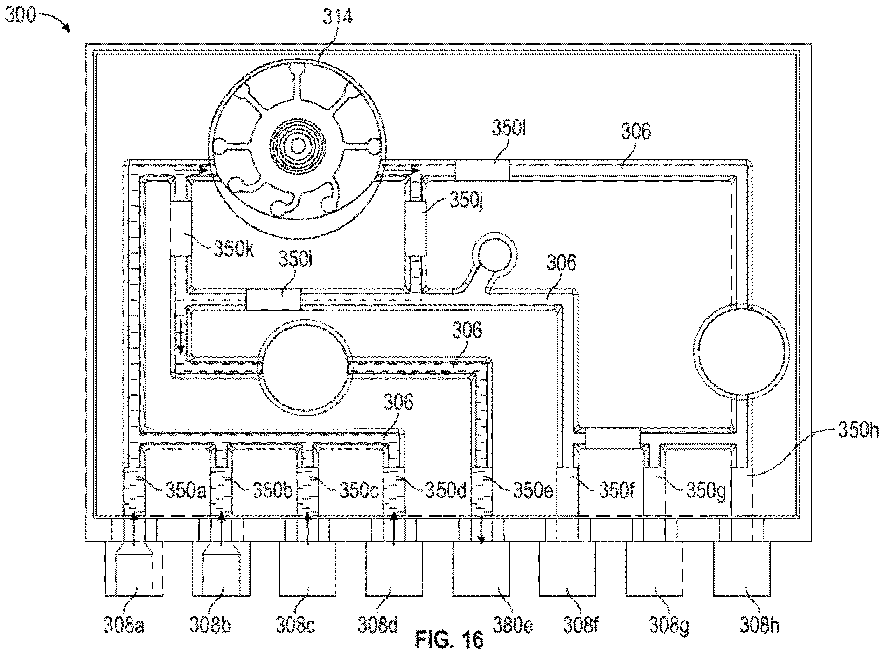

In FIG. 16, the cassette **300** can be in a filling configuration, in which the fill bag **124** is filled with the dialysis solution from one or more of the storage bags **122.** The valves **350a-e** are open, with the valves **350a-d** opening the connectors **308a-d** to the storage bags **122** and the valve **350e** opening the connector **308e** to the fill bag **124.** In addition, valves **350i** and **j** are also open and the pump assembly **214** can be operated in the first direction, which transfers the fluid from the storage bags **122** to the fill bag **124.**

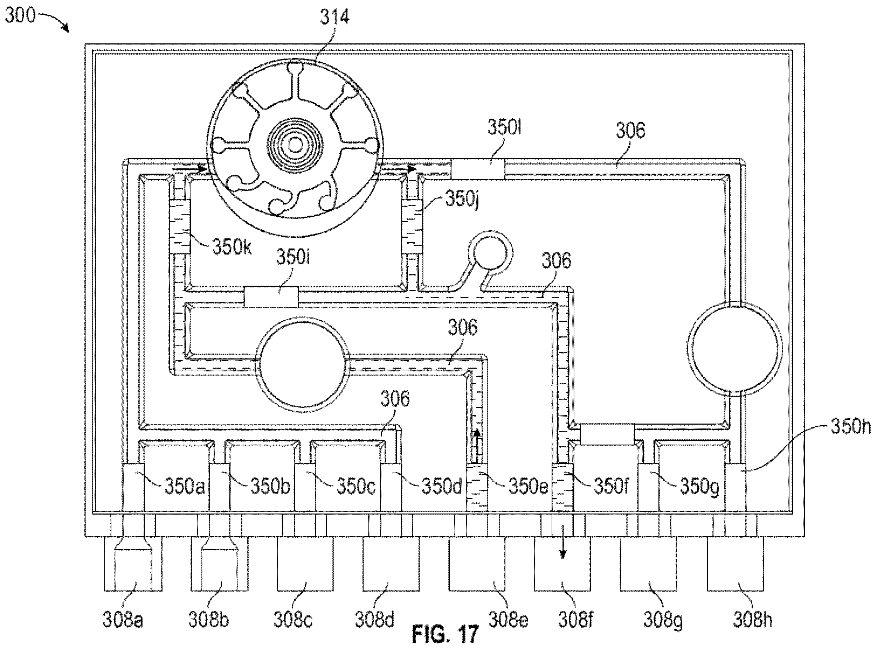

In FIG. 17, the cassette **300** can be in a priming configuration for priming the channels **306.** The valves **350e, f, k, j** are open and the pump assembly **214** is operated in a first direction such that dialysis solution from the fill bag line **128** connected to the connector **308e** flows to the connector **308f** and to the drain line **132.** The remaining valves **350a-d, g, h, i,** and **l** are closed. In this configuration, the fluid channels **306** interconnecting the connectors **308e** and **308f** are primed.

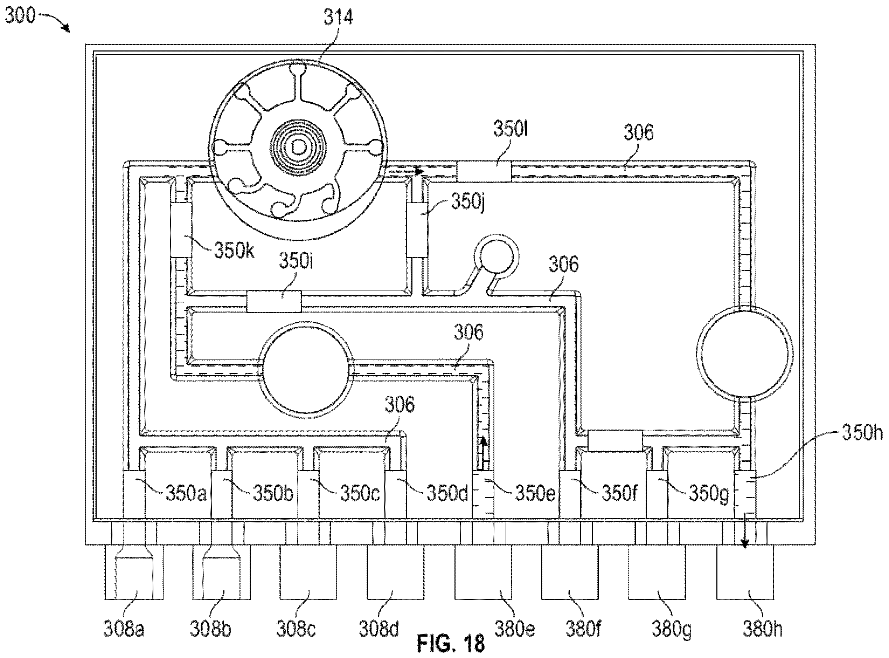

In FIG. 18, the cassette **300** is shown in a patient line priming and filling configuration, in which the valves **350e** and **h** are open and the pump assembly **314** is operated in the first direction to move the fluid to the patient lines **130.** In this configuration, the dialysis solution from the fill bag line **128** connected to the connector **308e** flows to the connector **308h** and to the patient line **130** to allow for the dialysis solution to be infused into the patient.

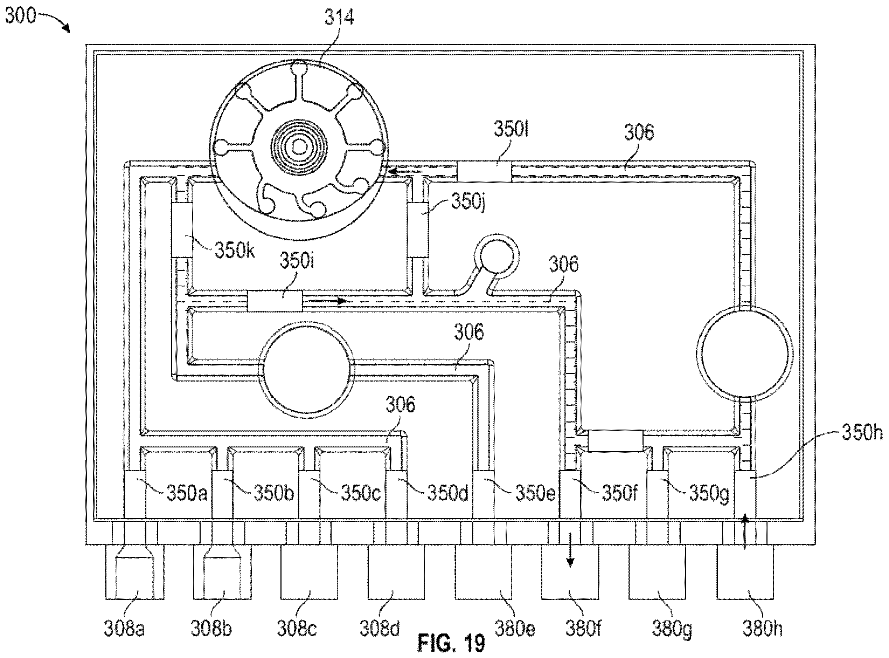

FIG. 19 shows the cassette **300** in a drain configuration, in which the dialysis solution can be removed from the patient after infusion is completed and the dialysis solution can have dwelled in the patient for a duration of treatment time. The valves **350a-e** and **g** are closed and the valves **350f, h,** and **i-I** are open. The pump assembly **314** can be operated in a second direction, reverse of the first direction, such that the dialysis solution is drained from the patient. In this configuration, the fluid can be withdrawn from the patient through the patient line **130** connected to the connector **308h** and to the connector **308f** coupled to the drain line **132.**

FIG.'s 20 and 21 show an embodiment of a cassette **400,** which is similar to the cassette **300.** The cassette **400** includes a pair of connectors **410** and **412** configured to couple to a pump assembly **414** that is disposed within the cassette interface **110** of the cycler **102.** Thus, the cassette **400** does not include an integrated pump assembly **214** as described above with respect to the cassettes **200** and **300.** The pump assembly **414** can be substantially similar to the pump assembly **214** of FIG.'s 6 and 7 and also includes a flexible rotor disposed within a circular pump housing. The cassette **400** can be operated in the same manner as the cassette **300** as described above with respect to FIG.'s 16-19 during transfer, priming, filling, and drain.

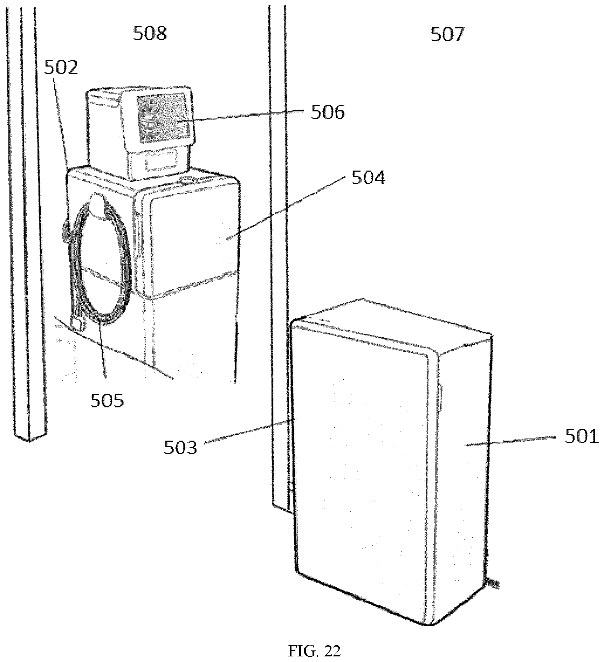

FIG. 22 illustrates a non-limiting embodiment of a preparator **501** and a cycler **502.** The preparator **501** can be connected to a water source (not shown), such as a tap water source of a house. Water can be flowed into the preparator **501** and purified to remove any contaminants in the water source. The preparator **501** can include dry powders, slurries, or liquid concentrates that are reconstituted or diluted with purified water to generate a desired concentrate or solution. The concentrates can be mixed and diluted to generate a peritoneal dialysis fluid. Door **503** in preparator **501** can be opened to obtain access to the components of the preparator **501,** the solutes used for generation of the peritoneal dialysis fluid, and for maintenance or replacement. In certain embodiments, a cassette, as described in any embodiment herein, can be included as part of the preparator **501** to direct fluid through the preparator to generate the peritoneal dialysis fluid.

Once the peritoneal dialysis fluid is generated by preparator **501,** the peritoneal dialysis fluid can be pumped to cycler **502** through fluid line **505** which can be connected to the preparator **501.** The fluid can be pumped into the cycler **502** and infused into the patient as described. In certain embodiments, one or more filters (not shown) can be included in the cycler **502** to remove bacteria and endotoxins from the peritoneal dialysis fluid prior to infusing the peritoneal dialysis fluid into a patient. A cassette, as described in any embodiment herein, can be included in the cycler **502** to direct fluid through the cycler **502,** filters, and into and out of the patient. Door **504** on cycler **502** can be included to allow access to the cassette and other components.

Graphical user interface **506** can be included on either or both of the preparator **501** and cycler **502.** Through the graphical user interface **506,** the user can control the water purification, peritoneal dialysis fluid generation, and treatment. The user can also receive messages and/or alerts. The cycler **502** can communicate with preparator **501** through any means known in the art, including Bluetooth, Wi-Fi, or wired communication to coordinate the water purification, peritoneal dialysis fluid generation, and treatment. In certain embodiments fluid line **505** can be long enough for preparator **501** and cycler **502** to be placed in separate rooms. As shown in FIG. 22, preparator **501** can be placed in a first room **507,** such as a bathroom, while cycler **502** is placed in a second room **508,** such as a bedroom. However, one of skill in the art will understand that a shorter fluid line can be used, and the modules placed closer together. Although shown as a single preparator **501,** the water purification and peritoneal dialysis fluid generation functions can be performed by separate connected modules.

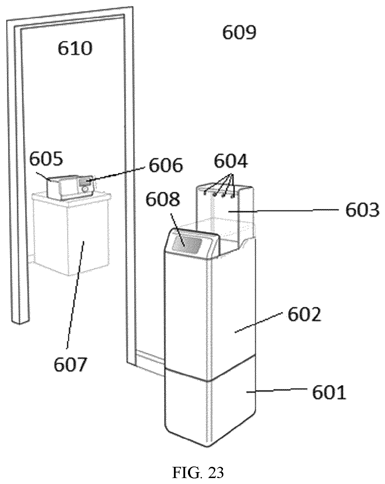

FIG. 23 illustrates an alternative arrangement of peritoneal dialysis components. As shown in FIG. 23, a preparator **602** can be connected to a water purification module **601.** Although shown as separate modules in FIG. 23, the preparator **602** and water purification module **601** can be a single module. As described, water from a water source (not shown) can be flowed through water purification module **601** and into preparator **602** to generate peritoneal dialysis fluid from dry powders and/or liquid concentrates. In the system illustrated in FIG. 23, the generated peritoneal dialysis fluid can be pumped into one or more peritoneal dialysis bags (not shown) that can be connected to preparator **602** and hung on hooks **604** in compartment **603.** In certain embodiments, compartment **603** can be collapsible, with the peritoneal dialysis bags inside the preparator **602** until removed by a user. A cassette, as described in any embodiment herein, can be included in the preparator **602** and/or water purification module **601** to direct fluid movement through the systems.

After generation of the peritoneal dialysis fluid, a user can remove a peritoneal dialysis fluid bag from hooks **604** and connect the peritoneal dialysis bag to cycler **605.** The cycler **605** can infuse the peritoneal dialysis fluid into the patient and drain used peritoneal dialysis fluid from the patient as needed. In certain embodiments, the cycler **605** can be small enough to fit on a tabletop **607.** In certain embodiments, one or more filters (not shown) can be included in the cycler **605** to remove bacteria and endotoxins from the peritoneal dialysis fluid prior to infusing the peritoneal dialysis fluid into a patient. A cassette, as described in any embodiment herein, can be included in the cycler **605** to direct fluid through the cycler **605,** filters, and into and out of the patient. As described, the preparator **602** and water purification module **601** can be placed in a first room **609,** such as a bathroom, while the cycler **605** can be placed in a second room **610,** such as a bedroom. User interface **608** on preparator **602** and/or user interface **606** on cycler **605** can be used to control the water purification, peritoneal dialysis fluid generation, and treatment.

## Claims

1. A dialysis cassette (200), comprising:
a cassette housing (201) formed from an inner portion (202) and an outer portion (204), wherein the entirety or a segment of the inner portion (202) is formed from a flexible membrane (203), the cassette housing (201) having a plurality of fluid channels (206) and a pump assembly (214) disposed within the cassette housing, the pump assembly (214) having a pump housing (218) and a flexible rotor (230) having a plurality of flexible vanes (236), wherein the flexible rotor (230) is rotatable in either a clockwise direction or a counterclockwise direction to move a defined amount of fluid through the plurality of channels (206) per each rotation of the flexible vanes (236); and
a plurality of valves (250) disposed within the plurality of channels (206),
**characterized in that** each valve (250) of the plurality of valves is a pinch valve having an undulating shape having two peak portions (252) with a deflectable valley portion (254) such that the valley portion (254) is thinner than the adjacent peak portions (252), and wherein each valve (250) has a bottom surface that abuts the flexible membrane (203).

2. The dialysis cassette according to claim 1, wherein the pump housing (218) has an inlet (224) and an outlet (226) coupled to the plurality of channels (206).

3. The dialysis cassette according to claim 2, wherein the pump housing (218) has a narrowing portion (228) disposed between the inlet (224) and the outlet (226).

4. The dialysis cassette according to claim 3, wherein the plurality of flexible vanes (236) is configured to compress when contacting the narrowing portion (228).

5. The dialysis cassette according to claim 4, wherein the flexible rotor (230) is comprised of an elastomer and a thermoplastic polymer.

6. The dialysis cassette according to claim 1, wherein the flexible rotor (230) is rotatable about a rotation axis perpendicular to a plane defined by the cassette housing (201).

7. The dialysis cassette according to claim 1, wherein the pump housing (218) has a drive shaft (240) and a gasket (225) disposed about the drive shaft, the gasket (225) configured to fluidly seal the pump housing (218).

8. The dialysis cassette according to claim 1, wherein the cassette housing (201) further has at least one sensor disposed in fluid contact with the plurality of channels (206).

9. The dialysis cassette according to claim 8, wherein the at least one sensor is at least one of a pressure sensor (270, 272), a temperature sensor (276), a conductivity sensor (274), an air bubble detector, or a flow sensor (278).

10. A dialysis system, comprising:
a dialysis cycler (102), a cassette interface (110), a patient line (130), a drain line (132); and a fluid source (122); and
a dialysis cassette (200) according to any of claims 1 to 9 configured to couple to the cassette interface (110), the patient line (130), the drain line (132), and the fluid source (122).

11. The dialysis system according to claim 10, wherein the cassette interface (110) has a plurality of actuators (260) configured to push on the deflectable valley portions (254) of the plurality of valves (250).

## Patentansprüche

1. Dialysekassette (200), umfassend:
ein Kassettengehäuse (201), das aus einem inneren Abschnitt (202) und einem äußeren Abschnitt (204) ausgebildet ist, wobei der gesamte innere Abschnitt (202) oder ein Teil davon aus einer flexiblen Membran (203) ausgebildet ist, das Kassettengehäuse (201) eine Vielzahl von Fluidkanälen (206) und eine Pumpenanordnung (214) aufweist, die innerhalb des Kassettengehäuses eingerichtet ist, die Pumpenanordnung (214) ein Pumpengehäuse (218) und einen flexiblen Rotor (230) aufweist, der eine Vielzahl von flexiblen Flügeln (236) aufweist, wobei der flexible Rotor (230) entweder im Uhrzeigersinn oder gegen den Uhrzeigersinn drehbar ist, um bei jeder Drehung der flexiblen Flügel (236) eine definierte Fluidmenge durch die Vielzahl von Kanälen (206) zu bewegen; und
eine Vielzahl von Ventilen (250), die innerhalb der Vielzahl von Kanälen (206) eingerichtet sind,
**dadurch gekennzeichnet, dass** jedes Ventil (250) der Vielzahl von Ventilen ein Quetschventil ist, das eine wellenförmige Form aufweist, das zwei Spitzenabschnitte (252) mit einem auslenkbaren Talabschnitt (254) aufweist, derart, dass der Talabschnitt (254) dünner ist als die angrenzenden Spitzenabschnitte (252), und wobei jedes Ventil (250) eine untere Oberfläche aufweist, die an die flexible Membran (203) angrenzt.

2. Dialysekassette nach Anspruch 1, wobei das Pumpengehäuse (218) einen Einlass (224) und einen Auslass (226) aufweist, die mit der Vielzahl von Kanälen (206) gekoppelt sind.

3. Dialysekassette nach Anspruch 2, wobei das Pumpengehäuse (218) einen Verengungsabschnitt (228) aufweist, der zwischen dem Einlass (224) und dem Auslass (226) eingerichtet ist.

4. Dialysekassette nach Anspruch 3, wobei die Vielzahl von flexiblen Flügeln (236) konfiguriert ist, um sich bei Kontakt mit dem Verengungsabschnitt (228) zusammendrücken.

5. Dialysekassette nach Anspruch 4, wobei der flexible Rotor (230) aus einem Elastomer und einem thermoplastischen Polymer besteht.

6. Dialysekassette nach Anspruch 1, wobei der flexible Rotor (230) um eine Drehachse drehbar ist, die senkrecht zu einer durch das Kassettengehäuse (201) definierten Ebene steht.

7. Dialysekassette nach Anspruch 1, wobei das Pumpengehäuse (218) eine Antriebswelle (240) und eine um die Antriebswelle eingerichtete Dichtung (225) aufweist, wobei die Dichtung (225) konfiguriert ist, um das Pumpengehäuse (218) fluidisch abzudichten.

8. Dialysekassette nach Anspruch 1, wobei das Kassettengehäuse (201) ferner mindestens einen Sensor aufweist, der in Fluidkontakt mit der Vielzahl von Kanälen (206) eingerichtet ist.

9. Dialysekassette nach Anspruch 8, wobei der mindestens eine Sensor mindestens einer von einem Drucksensor (270, 272), einem Temperatursensor (276), einem Leitfähigkeitssensor (274), einem Luftblasendetektor oder einem Durchflusssensor (278) ist.

10. Dialysesystem, umfassend:
einen Dialyse-Cycler (102), eine Kassettenschnittstelle (110), eine Patientenleitung (130), eine Abflussleitung (132); und eine Fluidquelle (122); und
eine Dialysekassette (200) nach einem der Ansprüche 1 bis 9, die konfiguriert ist, um mit der Kassettenschnittstelle (110), der Patientenleitung (130), der Abflussleitung (132) und der Fluidquelle (122) zu koppeln.

11. Dialysesystem nach Anspruch 10, wobei die Kassettenschnittstelle (110) eine Vielzahl von Aktuatoren (260) aufweist, die konfiguriert sind, um auf die auslenkbaren Talabschnitte (254) der Vielzahl von Ventilen (250) zu drücken.

## Revendications

1. Cassette de dialyse (200), comprenant :
un boîtier de cassette (201) formé d'une partie intérieure (202) et d'une partie extérieure (204), dans laquelle la totalité ou un segment de la partie intérieure (202) est formé d'une membrane flexible (203), le boîtier de cassette (201) ayant une pluralité de canaux de fluide (206) et un ensemble pompe (214) disposé à l'intérieur du boîtier de cassette, l'ensemble pompe (214) ayant un boîtier de pompe (218) et un rotor flexible (230) ayant une pluralité d'aubes flexibles (236), dans laquelle le rotor flexible (230) peut tourner soit dans un sens horaire, soit dans un sens antihoraire pour déplacer une quantité définie de fluide à travers la pluralité de canaux (206) à chaque rotation des aubes flexibles (236) ; et
une pluralité de soupapes (250) disposées à l'intérieur de la pluralité de canaux (206),
**caractérisée en ce que** chaque soupape (250) de la pluralité de soupapes est une soupape à pincement ayant une forme ondulée ayant deux parties crête (252) avec une partie vallée (254) déformable de telle sorte que la partie vallée (254) est plus mince que les parties crête (252) adjacentes, et dans laquelle chaque soupape (250) a une surface de fond qui vient en butée contre la membrane flexible (203).

2. Cassette de dialyse selon la revendication 1, dans laquelle le boîtier de pompe (218) a une entrée (224) et une sortie (226) accouplées à la pluralité de canaux (206).

3. Cassette de dialyse selon la revendication 2, dans laquelle le boîtier de pompe (218) a une partie rétrécie (228) disposée entre l'entrée (224) et la sortie (226).

4. Cassette de dialyse selon la revendication 3, dans laquelle la pluralité d'aubes flexibles (236) est conçue pour se comprimer lorsqu'elle est en contact avec la partie rétrécie (228).

5. Cassette de dialyse selon la revendication 4, dans laquelle le rotor flexible (230) est constitué d'un élastomère et d'un polymère thermoplastique.

6. Cassette de dialyse selon la revendication 1, dans laquelle le rotor flexible (230) peut tourner autour d'un axe de rotation perpendiculaire à un plan défini par le boîtier de cassette (201).

7. Cassette de dialyse selon la revendication 1, dans laquelle le boîtier de pompe (218) a un arbre d'entraînement (240) et un joint d'étanchéité (225) disposé autour de l'arbre d'entraînement, le joint d'étanchéité (225) étant conçu pour sceller de manière fluidique le boîtier de pompe (218).

8. Cassette de dialyse selon la revendication 1, dans laquelle le boîtier de cassette (201) a en outre au moins un capteur disposé en contact fluidique avec la pluralité de canaux (206).

9. Cassette de dialyse selon la revendication 8, dans laquelle l'au moins un capteur est au moins l'un parmi un capteur de pression (270, 272), un capteur de température (276), un capteur de conductivité (274), un détecteur de bulles d'air, ou un capteur de débit (278).

10. Système de dialyse comprenant :
un cycleur de dialyse (102), une interface de cassette (110), une ligne patient (130), une ligne de drainage (132) ; et une source de fluide (122) ; et
une cassette de dialyse (200) selon l'une quelconque des revendications 1 à 9, conçue pour s'accoupler à l'interface de cassette (110), à la ligne patient (130), à la ligne de drainage (132) et à la source de fluide (122).

11. Système de dialyse selon la revendication 10, dans lequel l'interface de cassette (110) a une pluralité d'actionneurs (260) configurés pour pousser sur les parties de vallée (254) déformables de la pluralité de soupapes (250).
